# EUROPEAN PATENT SPECIFICATION

(11) **EP 1 244 444 B1**
(45) Date of publication and mention of the grant of the patent: **29.12.2004**
(21) Application number: 00974020.0
(22) Date of filing: 27.10.2000
(51) Int. Cl.: A61K 31/407, A61P 31/04, C07D 487/04, C07D 477/20, A61K 31/40, C07D 477/02

(54) **PROCESS FOR FORMULATION OF CARBAPENEM ANTIBIOTIC COMPOSITIONS**
VERFAHREN ZUR HERSTELLUNG VON CARBAPENEM-ANTIBIOTIKA ZUBEREITUNGEN
PROCEDE DE PREPARATION DE COMPOSITIONS ANTIBIOTIQUES A BASE DE CARBAPENEM

(30) Priority: 29.10.1999 US 162482 P
(43) Date of publication of application: 02.10.2002
(73) Proprietor: Merck & Co., Inc., Rahway, New Jersey 07065-0907 (US)
(72) Inventor: AL-DEHNEH, Anthony, Rahway, NJ 07065-0907 (US); HUNKE, William, A., Rahway, NJ 07065-0907 (US); ILLIG, Kathleen, J., Rahway, NJ 07065-0907 (US); KANIKE, Anand, Rahway, NJ 07065-0907 (US); PATEL, Hiren, Rahway, NJ 07065-0907 (US); REYNOLDS, Scott, D., Rahway, NJ 07065-0907 (US); TSINONTIDES, Stelios, C., Rahway, NJ 07065-0907 (US)
(74) Representative: Horgan, James Michael Frederic
(86) International application number: PCT/US2000/029869
(87) International publication number: WO 2001/032172

(56) References cited:
- WO-A-98/18800
- US-A- 5 952 323
- ALMARSSON ET AL.: "Meropenem exists in equilibrium with carbon dioxide adduct in bicarbonate solution" J. PHARM. SCI., vol. 87, no. 5, 1998, pages 663-666, XP002235056
- SMITH PETER A.S. THE CHEMISTRY OF OPEN-CHAIN ORGANIC NITROGEN COMPOUNDS vol. 1, 1965, NEW YORK: W.A. BENJAMIN, page 263, XP002938614

## Description

Betalactams, a broader class of antibiotics, further defined as carbapenems useful for the treatment of infectious diseases, including gram positive and negative, aerobic and anaerobic bacteria. U.S. Application Nos. 08/926,915 and 09/060,691 to Almarsson *et al*, filed September 10, 1997, and April 15, 1998, respectively, now assigned to Merck & Co., Inc., teach a novel carbapenem antibiotic compound of formula I: 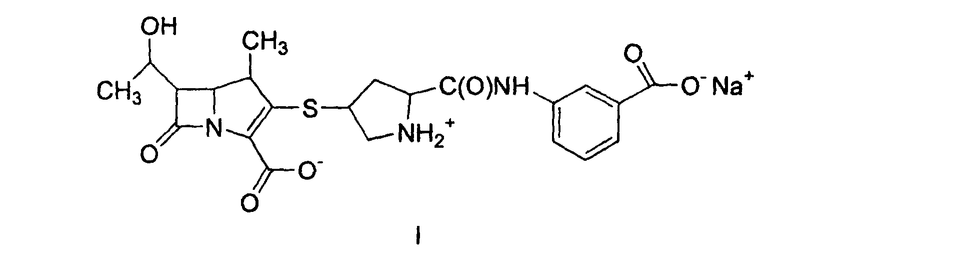 and a process for the preparation thereof. The compound of formula I, prepared by chemical synthesis, is a relatively unstable, monosodium salt at ambient conditions, i.e. 20°C and 1 atmosphere, and remains unstable at temperatures above about -20°C, wherein it undergoes dimerization and hydrolysis to form undesirable dimers and open ring by-products. Almarsson suggests a method of carbonation to converting the compound of formula I to a stable compound of formula II: 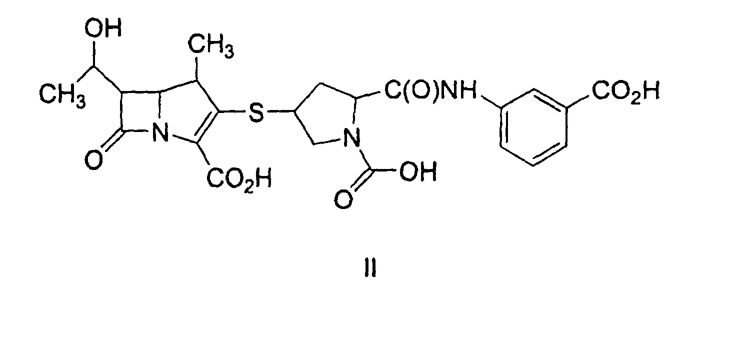 The method of stabilization requires the use of carbon dioxide, i.e. potassium, magnesium, calcium or sodium carbonates and bicarbonates as suitable carbon dioxide sources, and water or saline solution as a suitable solvent to produce the compound of formula II.

U.S. Patent No. 5,952,323, to *Zimmerman et al*, issued September 14, 1999, assigned to Merck & Co., Inc., teaches a more detailed process for converting the carbapenem monosodium salt into a stable carbapenem carbon dioxide adduct. In accordance with Zimmerman, specific mole ratios of sodium carbonate and sodium bicarbonate to unstabilized carbapenem monosodium salt, as well as pH limitations are suggested. The reference also provides solubility data for intravenous formulation at fixed conditions.

While Almarsson teaches reaction synthesis and conditions for preparation of the bulk carbapenem of formula I, and Zimmerman suggests CO₂ concentrations, pH and solubility ranges, neither reference provide a detailed step-by-step method for preparing a carbapenem formulation containing a stabilized CO₂ adduct of formula II. Due to the instability of the compound at temperatures above about -20°C, as well as its sensitivity to pH fluctuation, processing conditions for converting the bulk drug of formula I to the formulation of formula II are critical to providing a sterile, finish product of high quality.

It is now desirable to provide a process for converting a bulk drug, - unstabilized carbapenem antibiotic, requiring storage at low temperatures, to a stabilized, carbapenem antibiotic, formulation suitable for intravenous and intramuscular injection into a patient in need thereof. It is also desirable to provide a product with needed solid state stability at room temperature and reconstitution stability for dosing.

### SUMMARY OF THE INVENTION

The present invention is directed to a novel process for converting an unstablized, monosodium adduct of carbapenem of formula I into a stabilized, carbon dioxide adduct of carbapenem for the treatment of bacterial infections in mammal patients, comprising the steps of : 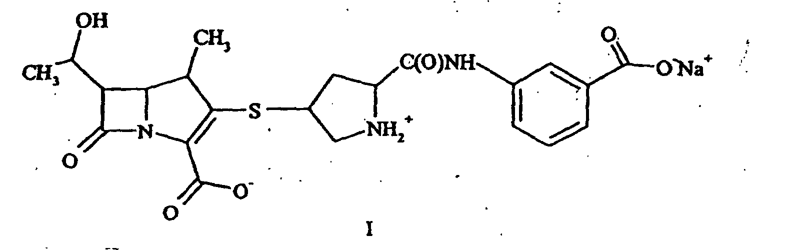
a. preparing from about I to about 3N solution of sodium hydroxide, chilling the solution to a temperature of from 0° to 10°C;
b. charging from 40 to 60% by wt., based on 100% by wt. total batch weight, of Water for Injection into a compounder having means for mixing, and cooling the water to a temperature of from 0° to 10°C;
c. charging 1 mole equivalent of carbonates/active beta-lactam, wherein the carbonate are selected from sodium bicarbonate, sodium carbonate and mixtures thereof, into the compounder while mixing, to prepare a carbonate solution, while maintaining a temperature of from 0° to 10°C;
d. maintaining the carbonate solution at a temperature range of from 0° to 10°C, and a pH of from 7.5 to 9.0;
e. thawing a sufficient amount of unstabilized, beta-lactam from a temperature of -20°C to a temperature of from 5° to 25°C to prepare a final, formulation containing about 200 g/liter of active beta-lactam, and charging at the same time into the compounder from 0.7 to 1.0 mole of sodium hydroxide/mole of active beta-lactam, while mixing the carbonate solution to dissolve the beta-lactam therein, and maintaining the compounder temperature of from 0° to 5°C to produce a beta-lactam-carbonate solution;
f. adding the sodium hydroxide solution to the beta-lactam-carbonate solution, as required, during step e. to maintain the pH of the solution of from 7.0 to 8.0.
g. adding water, as required, to adjusting the beta-lactam-carbonate solution to a range of 95 to 97 weight %, based on 100 total weight %, and maintaining a temperature of from 0° to 5°C;
h. adding the sodium hydroxide solution to the beta-lactam-carbonate solution, as required, to maintain the solution in a pH of from 7.2 to 7.8;
i. adding water, as required, to adjust the beta-lactam-carbonate solution to 100 weight % total, and maintaining the temperature of from 0° to 5°C;
j. sealing the compounder containing the beta-lactam-carbonate solution and pressurizing to from 0.7 bar (10 psig) to 2.1 bar (30 psig) to initialize filtration;
k. filtering the beta-lactam-carbonate solution through a sterilizing filter into a continuously cooled, sterile, receiving vessel exhibiting a temperature of from 0° to 5°C to produce a sterile, stabilized beta-lactam formulation;
l. aseptically filling the formulation into sterilized glass vials;
m. partially sealing the glass vials with dry, sterilized stoppers;
n. lyophilizing the solution by freezing in the glass vials at a temperature of from -45° to -40°C to produce a frozen formulation;
o. primary drying the frozen formulation at a temperature of from - 25 to -105°C for 48 to 60 hours at a pressure of about 0.1mbar (80mTorr) or lower;
p. secondary drying the formulation at a temperature from 40° to 60°C at pressure of about 0.1 mbar (80mTorr) or lower from about 3 to about 10 hours;
q. cooling the vials to ambient temperature; and
r. sealing the vials under partial vacuum, while maintaining a temperature of about 25°C.

### DESCRIPTION OF THE DRAWINGS

FIG. 1 is a graphical illustration of temperature and pH changes, and weight % of bulk drug addition during the compounding cycle of Example 1;
FIG. 2 is a graphical illustration of weight % of bulk monosodium salt-containing carbapenem added, active concentration, and mole ratio of sodium hydroxide (base) to mole of active carbapenem during the compounding cycle of Example 1;
FIG. 3 is a graphical illustration of the pressure and temperature changes during the lyophilization cycles of Examples 1 and 2;
FIG. 4 is a graphical illustration of the temperature and pH change, and weight % of bulk drug addition during the compounding cycle of Example 2;
FIG. 5 is a graphical illustration of weight % of bulk monosodium salt-containing carbapenem added, active concentration, and mole ratio of sodium hydroxide (base) during the compounding cycle of Example 2;

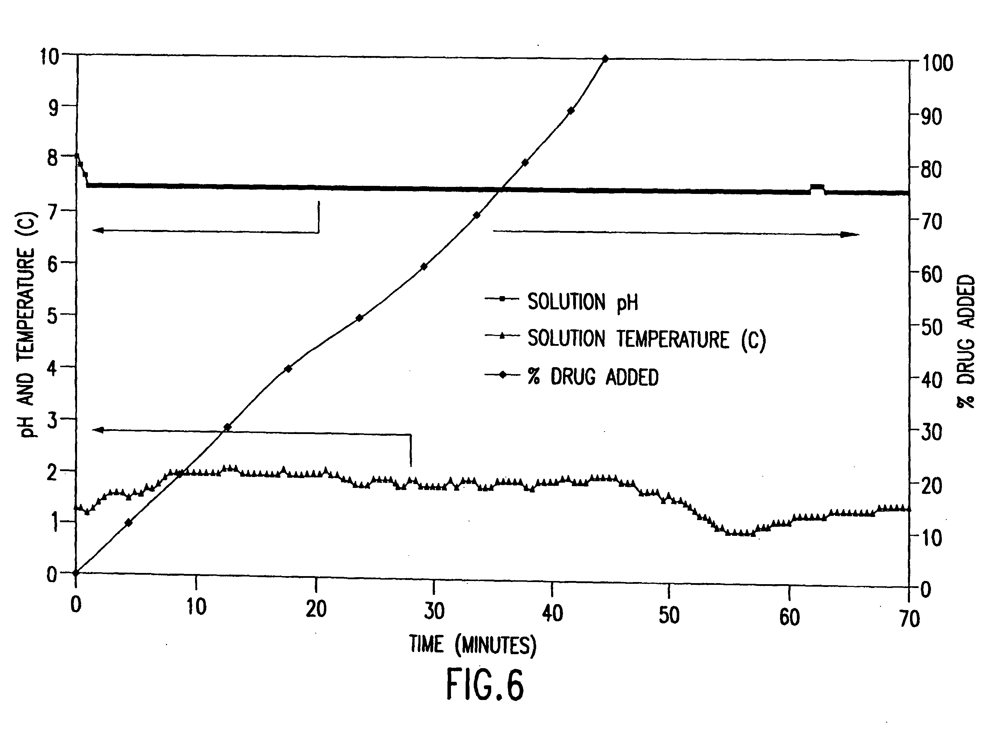

FIG. 6 is a graphical presentation of temperature and pH changes, and weight % of bulk drug addition during the compounding cycle of Example 3;

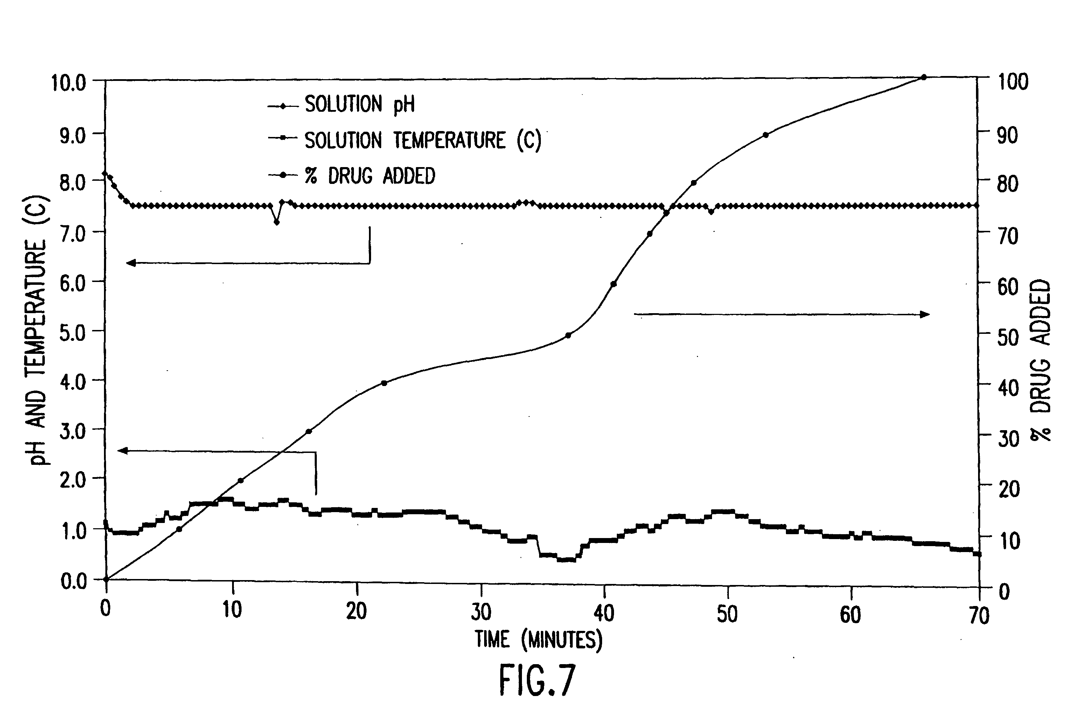

FIG. 7 is a graphical illustration of temperature and pH changes, and weight % of bulk drug addition during the compounding cycle of Example 4; and

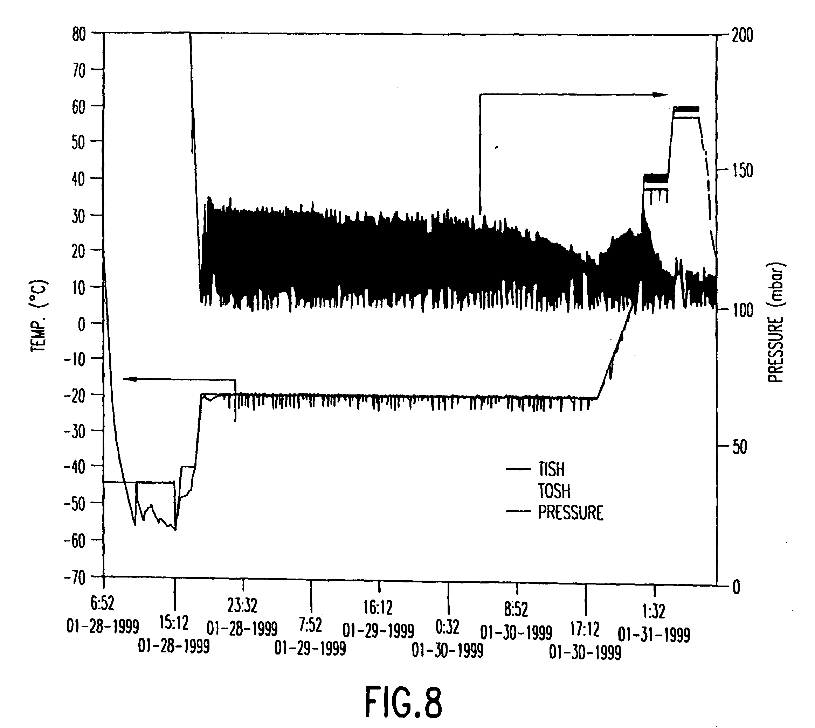

FIG. 8 is a graphical illustration of the pressure and temperature changes during the lyophilization cycles of Example 3; and

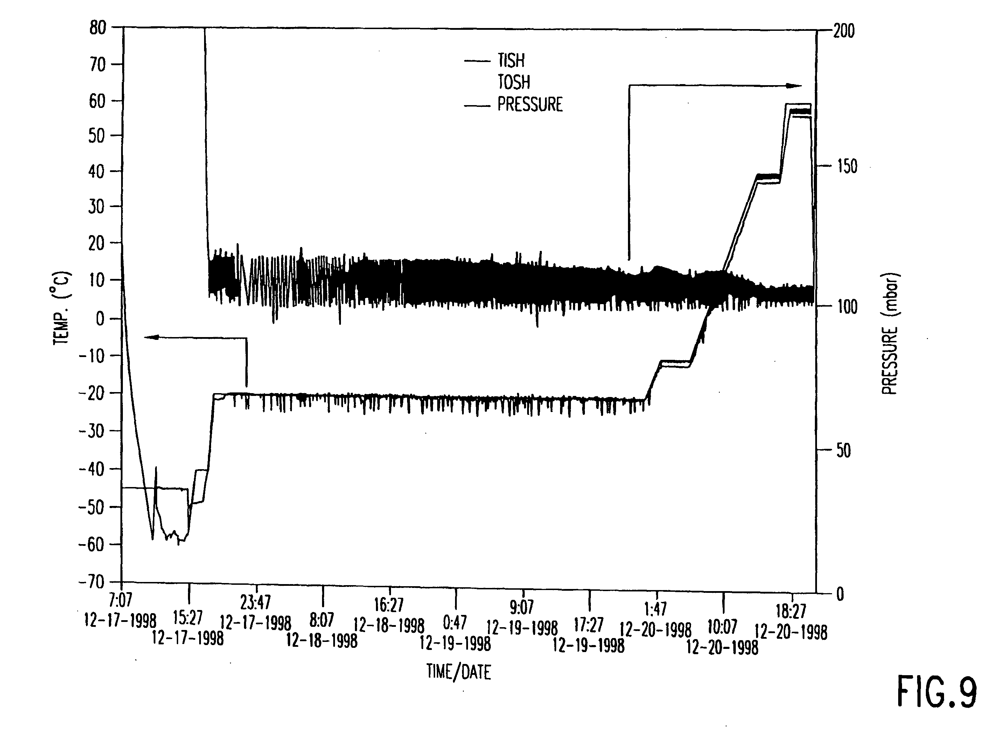

FIG. 9 is a graphical illustration of the pressure and temperature changes during the lyophilization cycle of Example 4.

### DETAILED DESCRIPTION OF THE INVENTION

As used herein, the term "1 mole equivalent" is defined as 1 mole of carbonates per 1 mole of active drug, wherein carbonates are selected from bicarbonates and carbonates, e.g. sodium bicarbonate, sodium carbonates, etc.

The term "bulk drug" "bulk active drug" or "bulk active beta-lactam" or "bulk active carbapenem," as use herein, is defined as the amount of actual unstable, beta-lactam, carbapenem and/or monosodium salt-containing carbapenem removed from cold storage.

The term "active drug," as used herein, is defined as the actual amount of beta-lactam, unstabilized and stabilized carbapenem, and monosodium salt-containing carbapenem and carbon dioxide-containing carbapenem. For example, the active drug is the amount of bulk drug less non-carbapenem, e.g. dimers and open ring by-products.

The term "*quantum sufficit*" ("q.s."), as used herein, is defined as the amount of a reagent necessary to increase the batch weight or volume to a specified total. As an example, a q.s. of 95% by wt. % means the amount of reagent required to bring the weight percent up to 95% by weight, based on 100% total weight.

The term "solid state stability", as used herein, is defined as the ability of finished, solid, lyophilized formulation (a porous off-white cake), at the end of about 2 years, to deliver the prescribed, labeled dosage of active drug.

The term "reconstitution stability", as used herein, is defined as the ability of a solution prepared by the finished, solid, lyophilized formulation into an appropriate diluent (i.e. 0.9% saline for injection, D5W, 1% Lidocaine, etc.) to deliver the prescribed, labeled dosage of active drug.

During the manufacture of bulk compound products such as beta-lactam and carbapenem antibiotics, the pharmaceutical compound is prepared by chemical synthesis from raw materials in large quantities. As with many carbapenem compounds, the compound in accordance with formula I is prepared in large batches as a monosodium salt. The compound is a weakly crystalline solid, hygroscopic at ambient conditions, and is unstable at room and refrigerated temperatures. Therefore, the bulk compound must be stored at a temperature of about -20°C to prevent degradation into dimer and open ring by-products.

The unstable carbapenem, after bulk manufacturing can be stored for long periods of time at -20°C and 1 atmosphere as a white powdery substance. However, this bulk compound must be converted into a stable formulation prior to use as once-a-day antimicrobial agent for intravenous (IV) and intramuscular (IM) administration.

The present invention is directed to a novel process for converting the unstable, monosodium salt of carbapenem antibiotic into stable, lyophilized carbon dioxide salt of carbapenem antibiotic that is suitable for the treatment of antibacterial infections in mammal patients. The references mention earlier herein address the bulk compound and method of preparing the carbon dioxide adduct, but fail to teach the conversion of the monosodium salt-containing compound to a formulation exhibiting acceptable levels of degradates required for solid state and reconstitution stability for dosing to patients.

The earlier mentioned references revealed that compound dimerization is inhibited via the formation of a reversible equilibrium adduct between carbon dioxide and monosodium salt of carbapenem compound of formula I, according to the following scheme: 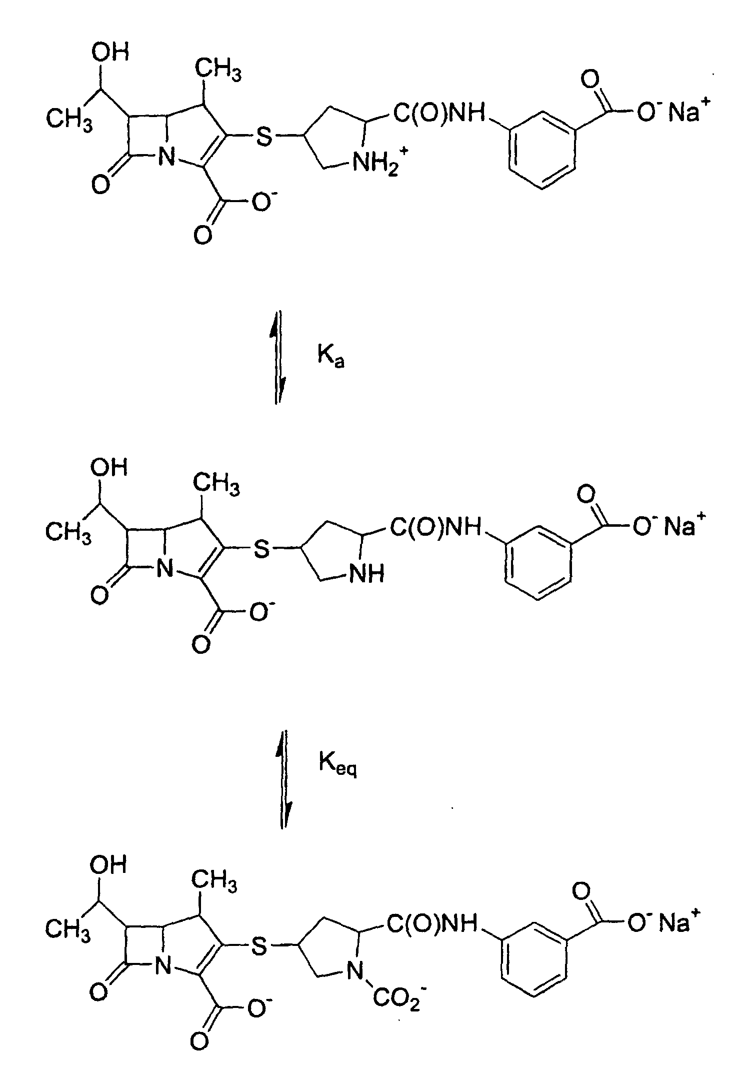 wherein Kₐ and K_{eq} are equilibrium constants of the reactions.

In accordance with the process of the present invention, unstable, carbapenem antibiotics are prepared into stabilized, carbapenem antibiotics suitable for the treatment of bacterial infections of patients in need thereof. More particular, the process converts unstable, monosodium salt of carbapenem antibiotic into stable, carbon dioxide adduct of carbapenem antibiotic formulation suitable for the treatment of bacterial infections in mammal patients. This sterile formulation can be administered intravenously or intramuscularly.

The batch-wise process of the invention, conducted under aseptic conditions, requires several reagents and processing units to prepare formulations of high quality, wherein the rate of conversion from the monosodium salt to the carbon dioxide adduct is high, and the formation of by-products, e.g. dimer and open ring compounds, is low. The mole ratio of sodium bicarbonate and sodium carbonate to active, bulk carbapenem, processing temperatures, pHs, mixing, and lyophilizing conditions are critical to the preparation of a formulation of high pharmaceutical quality.

The process for preparing a stable intravenous formulation of a carbon dioxide adduct of a carbapenem requires the processing temperature to be maintained within the range of about 0° to about 5°C and the pH of the pre-lyophilized, active solution to be maintained within the range of about 7.0 to about 8.0. The process is conducted under aseptic conditions. All reagents utilized during the processes described herein meet *United States Pharmacopeia and National Formulary* standards unless otherwise noted.

### The Reagents

Water for Injection *United States Pharmacopeia* (USP), H₂O, (hereinafter referred to as "WFI"), water purified by distillation or reverse osmosis having a molecular weight of 18.02 (CAS-7732-18-5), is utilized herein as a pharmaceutical solvent.

Sodium Hydroxide *National Formulary* (NF), NaOH, purified sodium hydroxide having a molecular weight of 40.00 (CAS-1310-73-2), is utilized herein as a pharmaceutical aid to control the pH of the reagents in the compounder/reactor. Generally, the pH is maintained in the alkaline region, e.g. pH of about 7.0 to about 9.0, throughout the cycling time of the process.

Sodium Bicarbonate *United States Pharmacopeia* (USP), NaHCO₃, purified carbonic acid monosodium salt having a molecular weight of 84.01 (CAS-144-55-8), is utilized herein as a primary source of alkalizing agent, i.e. carbonate.

Sodium Carbonate *United States Pharmacopeia* (USP), Na₂CO₃, purifed carbonic acid, disodium salt or disodium carbonate having a molecular weight of 105.99 (CAS-497-19-8), is utilized herein as a second source of alkalizing agent, i.e. carbonate.

### The Process

Initially, a normal solution of from about 1 to about 3N sodium hydroxide is prepared by dissolving a sufficient amount of sodium hydroxide NF pellets in a sufficient amount of Water For Injection, USP. While adding the sodium hydroxide, the solution is constantly mixed to ensure complete dissolution. The compounder or reactor (up to 200L stainless steel jacketed vessel) utilized in the process is jacketed and cooled to maintain low temperatures and prevent bulk drug degradation during the process, and a variable agitation system is attached to the compounder to promote complete dissolution of the bulk drug into solution. Generally, about 40% by weight or 60% by volume of WFI is charged into the compounder to begin the process, and the water is cooled to the target temperature range of about 1° to about 5°C. To measure the pH of the solution in the compounder, appropriate pH and temperature devices are utilized; the pH meter is typically calibrated with buffers of pH 7.0 and 10.0. To control the pH during the batch-wise process, an appropriate pH Controller system equipped with a pump is utilized to meter NaOH solution into the compounder to maintain the pH within the required range.

After the WFI in the compounder is cooled, mixing is commenced to prevent localization of pH, temperature and concentration of reagents and bulk antibiotic drug. Sodium bicarbonate and/or sodium carbonate USP, in an amount sufficient to provide a final, formulation concentration thereof of about a 1 mole equivalent (defined above) is slowly added to the compounder under continuous mixing of the WFI. This solution is mixed until the carbonates are completely dissolved and the general pH of the solution is measured to ensure that it is between about 7.5 and about 9.0, preferably about 8.3, at a temperature in the range of about 0° to about 5°C, preferably about 2°C. The temperature and pH of the solution must be confirmed prior to beginning the addition of bulk drug. The unstable, bulk carbapenem drug is removed from a refrigerated unit held at about -20°C and thawed to a temperature of from about 5° to about 25°C for about 1 hour. A sufficient amount of the bulk drug is weighted out to provide a final formulation concentration of carbapenem of about 200g of active drug (as free acid)/liter formulation. During the addition of the bulk active carbapenem to the compounder, the carbonate solution is constantly mixed. Generally, the mixing will begin at lower revolutions during the initial addition of bulk drug to the solution and as the amount of bulk in the solution is increased, the rpm of mixing is increased proportionally thereto. The primary purpose of mixing is to ensure complete dissolution of the bulk drug into the solution. As necessary, sodium hydroxide solution is added to the compounder during the addition of the bulk drug to maintain the solution within the target pH of about 7.0 to about 8.0, preferably a pH of about 7.5. The bulk drug is generally slowly added to the compounder at about a constant rate over a time period of from about 30 to about 90 minutes to enhance dissolution. At the end of the bulk drug addition, the solution is mixed for an additional time of about 5 minutes and complete dissolution thereof is confirmed. The q.s. of the batch weight is adjusted to about 95% by weight of the final weight of the formulation with WFI, if needed, wherein the temperature is maintained between about 0° and about 5°C. Further NaOH titration is performed over a 10 to 20 minute period to ensure a mole ratio of NaOH/bulk, active drug in the range of from about 0.7 to about 1.0, typically from about 0.75 to about 0.95, and preferably from about 0.8 to about 0.9. Finally, the batch is adjusted to 100% by weight of its final weight with WFI with moderate mixing.

Afterwards, the solution is filtered through a sterilizing filter of from about 0.2 to about 0.25µm. When making larger batches, generally from about 10 to about 200 liters in a compounder, the compounding vessel is sealed and pressurized to initiate filtration. Filtration can be done either by pumping the solution through sterilizing filters with an appropriate pump in the absence of compounding vessel that can be pressurized or appropriate gas to carry out filtration by pressure. The receiving vessel for the filter formulation should be sterile and cooled to a temperature in the range of from about 0° to about 5°C. The filtered, formulation solution density will generally be from about 1.0 to about 1.2 g/ml at 0-5°C, typically about 1.1g/ml. Next, the filtered formulation is filled into vials and partially sealed with dry, sterile siliconized lyophilization stoppers. In the following examples conventional 20ml vials and 15ml ADD-Vantage™ vials are utilized. The filled vials are placed onto lyophilizer shelves pre-cooled to a temperature of from about -40° to about -45°C, typically about -40°C.

The lyophilization cycles used herein for the different vials are described in the examples, below. Generally, the cycle requires the vials to be soaked at about -40°C for about 2 hours and then heated to a temperature in the range of from about -25° to about -15°C shelf temperature at a rate of about 0.5°C/minute. The temperature is normally held in a range of from about -25° to about -15°C, and the lyophilizer chamber pressure held at about 80mTorr for a time period of from about 48 to about 60 hours. The vials are heated to about 10°C shelf temperature at a rate of about 0.1 °C/minute and then to about 40°C shelf temperature at a rate of about 0.5°C/minute and held at 40°C for up to about 3 hours at a pressure of about 80mTorr or lower. Lastly, the vials are heated to about 60°C shelf temperature at a rate of about 0.5°C/minute and held there at 80mTorr or less for a time period of from about 3 to about 10 hours, and the shelves are cooled to ambient temperature (about 20° to about 30°C). The vials are completely sealed under a partial vacuum of about 0.9bar/700Torr or lower before removing them from the lyophilizer. Finally, the vials are stored at a temperature not exceeding about 25°C until needed.

In accordance with one preferred embodiment of the invention, there is described a novel process for preparing an intravenous formulation suitable for the treatment of bacterial infection, characterized as converting an unstabilized, carbapenem compound of formula I: 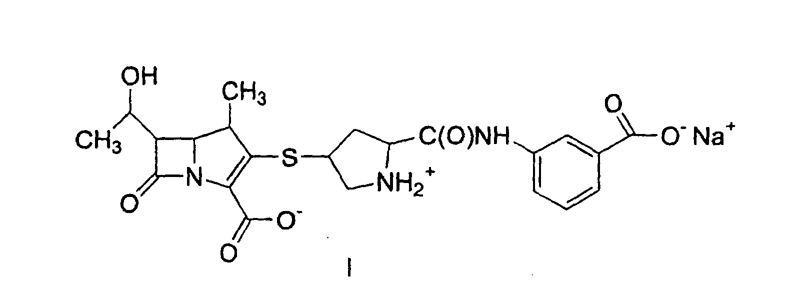 into a stabilized, carbapenem compound of formula II: 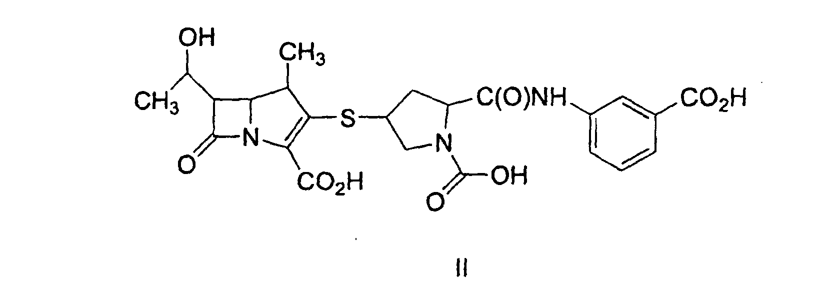 comprising the steps of:
a. preparing a solution of from about 1 to about 3N of sodium hydroxide and chilling the solution to a temperature of from 0° to 5°C;
b. charging a total of from 40 to 60% by wt., based on 100% by wt. total of the batch weight of Water for Injection exhibiting a temperature of from 2° to 85°C into a compounder having means for mixing, and cooling the water to a temperature of from 0° to 10°C;
c. charging sufficient carbonate selected from sodium bicarbonate, sodium carbonate and mixtures thereof, into the compounder to prepare a final formulation exhibiting about 1 mole equivalent of carbonates/active carbapenem , dissolving the same, and maintaining the solution at a temperature range of from 0° to 5°C to prepare a carbonate solution;
d. maintaining the carbonate solution at a pH of from 7.5 to 9.0, and a temperature of 0° to 5°C;
e. thawing a sufficient amount of bulk carbapenem of formula I to provide a formulation exhibiting a concentration of about 200 g (as free acid)/l from a temperature of -20°C to temperature of from 5 to 25°C, and slowly charging the same into the compounder with mixing of the carbonate solution to completely dissolve the bulk compound, while maintaining the compounder temperature of from 0° to 5°C to produce an active carbapenem solution;
f. simultaneously adding the an about 1 to about 3N sodium hydroxide solution to the active carbapenem, as required during step e., and maintaining a pH of from 7.0 to 8.0;
g. adjusting the active carbapenem solution to about 95% by weight of the final product weight, based on 100 total weight percent, utilizing water for injection, as required and maintaining the bulk carbapenem solution in a temperature of from 0° to 5°C;
h. adding the about 1 to about 3N sodium hydroxide solution to the bulk carbapenem solution to maintain a pH of from 7.2 to 7.8, as required;
i. adding Water for Injection, as required, to adjust the active carbapenem solution of 100% by wt., based 100% by wt. total weight, and maintaining a temperature of from 0 to 5°C;
j. sealing and pressurizing the compounder containing the bulk compound solution to a pressure of about 1 bar (15 psig) to initiate filtration;
k. filtering the bulk compound solution through a sterilizing filter into a continuously cooled, sterile, receiving vessel exhibiting a temperature of from 0° to 5°C; and
l. aseptically filling the intravenous formulation into sterile glass vials, and partially sealing the vials with dry, sterilized, lyophilization stoppers;
m. lyophilizing the formulation by freezing in the glass vials at a temperature of from -45° to -40°C to produce a frozen formulation;
n. primary drying the frozen formulation at a temperature of from -25° to -15°C for 48 to 60 hours at a pressure of about 0.1 mbar (80mTorr) or lower;
o. secondary drying the formulation at a temperature of from 40° to 60°C at a pressure of about 0.1mbar 80mTorr or lower for a time period of from 3 to about 10 hours;
p. cooling the vials to ambient temperature; and
q. sealing the vials under a partial vacuum of about 0.9 bar/700Torr or lower, while maintaining a temperature of about 25°C,
wherein the stabilized, carbapenem antibiotic formulation of formula II exhibits a carbapenem concentration of about 200g/l and a carbonate content of about 1 mole equivalent.

The following examples are provided for illustrative purposes and should not be construed as limiting the invention disclosed herein.

### Example 1

At ambient temperature and pressure, a 2N sodium hydroxide solution was prepared by dissolving 20g of sodium hydroxide NF pellets in 250ml of water for injection (WFI) while mixing. A Beckman pH probe was calibrated using pH 7 and 10 buffers. Into a Kontes 317000-1000, one (1) liter glass, compounder/reactor vessel with jacketed cooler and agitator was charged 400ml of WFI (about 50% of total batch volume), which was cooled to 5°C. Thereafter, 28.0g of sodium bicarbonate USP were dissolved into the compounder, and the compounder was held at a temperature of between 1° and 5°C, and a pH of between 8.1 and 8.5.

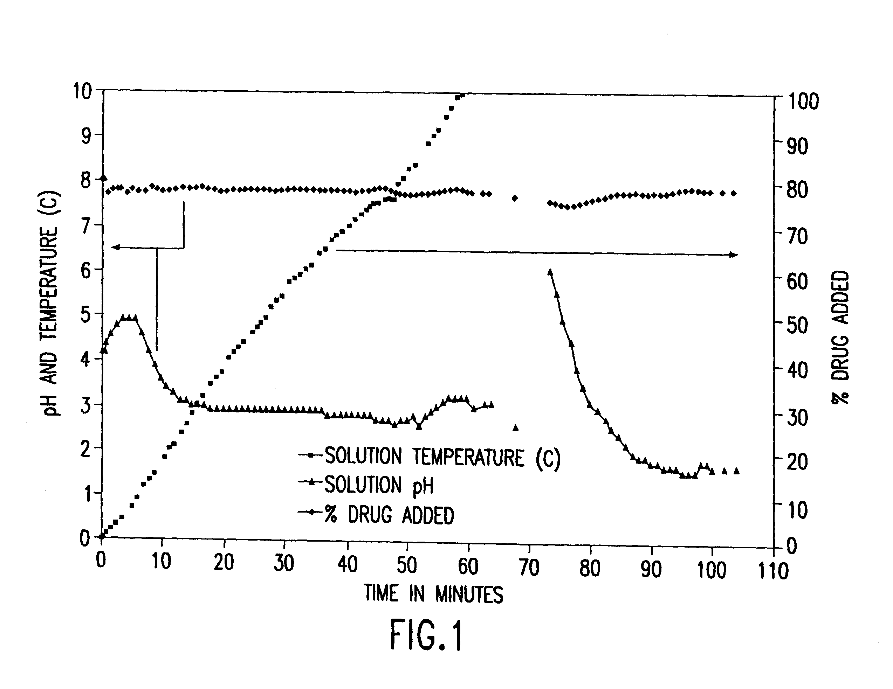

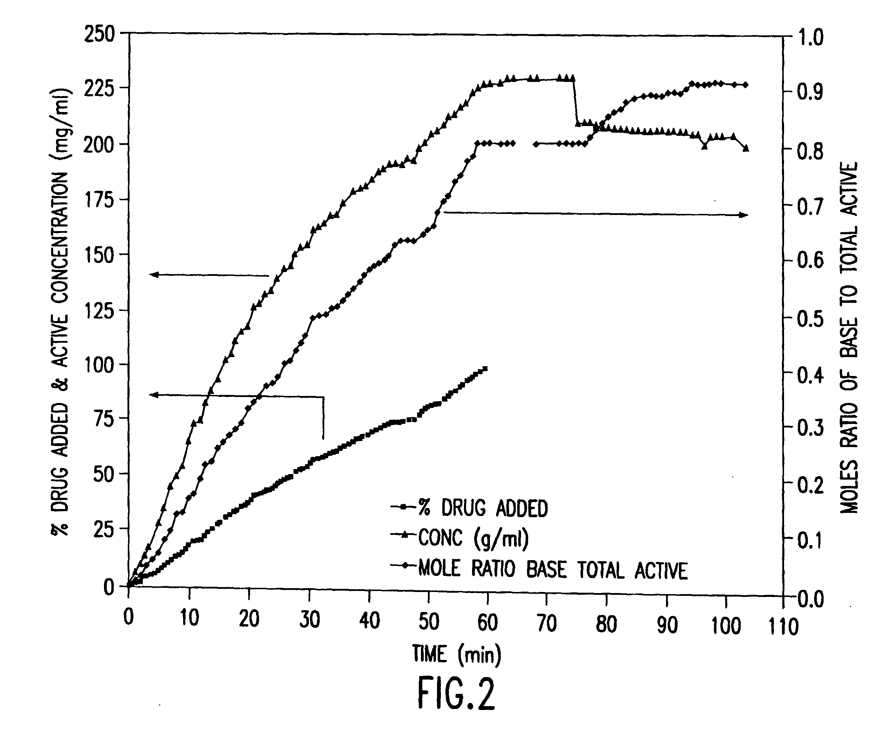

Unstable carbapenem, bulk drug substance, 160g of free acid exhibiting a moisture content of about 17.0% by weight, was thawed to room temperature from -20°C for 30 minutes. To reduce localization of pH, the 2N solution of sodium hydroxide was metered sub-surface into the compounder by a Masterflex peristaltic pump through size 16 tubing and a one (1) foot long x 1/16 inches diameter stainless steel dip tube. The bulk drug was divided into ten (10) equal portions and gradually added to the sodium bicarbonate solution over a 60 minutes period to ensure complete dissolution. FIG. 1 depicts the pH and temperature fluctuations during the bulk drug addition to the compounder. During the addition of the bulk drug, the sodium bicarbonate solution was constantly agitated. The solution temperature was maintained between 1° and 6°C and the pH at a set point of 7.8 by the addition of sodium hydroxide solution (see FIG. 1). Following the addition of the bulk drug, the batch weight was adjusted to 95% of the final weight with WFI maintained at a temperature of 1° to 5°C to produce a bulk drug-sodium bicarbonate solution. While the bulk drug-sodium bicarbonate solution was agitated for an additional 20 minutes, 2N sodium hydroxide titrations were performed to achieve a mole ratio of sodium hydroxide to bulk drug of 0.93. The final weight of the batch was adjusted to 100% total with chilled WFI at 1° to 5°C with additional agitation for 5 minutes. The total drug addition and compounding time was 102 minutes, and final batch weight was 888.0g. FIG. 2 provides the bulk drug concentration, % bulk drug added during compounding, and the mole ratio of base (H) to total active drug, wherein "total active drug" is the concentration of carbapenem within the batch.

While maintaining the solution at a temperature between 1° and 5°C, the bulk drug-sodium bicarbonate solution was filtered utilizing a Sterivex GV filter unit containing a 0.22µm filter into a sterile plastic container using a peristaltic pump. Immediately thereafter, 6.33g of the solution was placed into conventional 20ml vials utilizing a manual filler, and the vials were frozen to -70°C. The vials were partially stoppered and placed onto the shelves of a Virtis Lyophilizer pre-cooled to -40°C. Thereafter, the lyophilizer was operated according to the following cycle:
i) soak at -40°C shelf temperature for 2hrs;
ii) heat to -20°C shelf temperature at rate of 0.5°C/min;
iii) hold shelf temperature at -20°C and 80mTorr pressure for 48 hrs;
iii) heat to 10°C shelf temperature at rate of 0.1°C/min;
iv) heat to 40°C shelf temperature at rate of 0.5°C/min;
v) hold at 40°C and 80mTorr for 3hrs;
vi) heat to 60°C shelf temperature at rate of 0.5°C/min;
vii) hold at 60°C and 80mTorr for 3hrs;
viii) cool the shelves to ambient temperature (20°-30°C); and
ix) stopper under partial vacuum of 0.9 bar/700Torr.

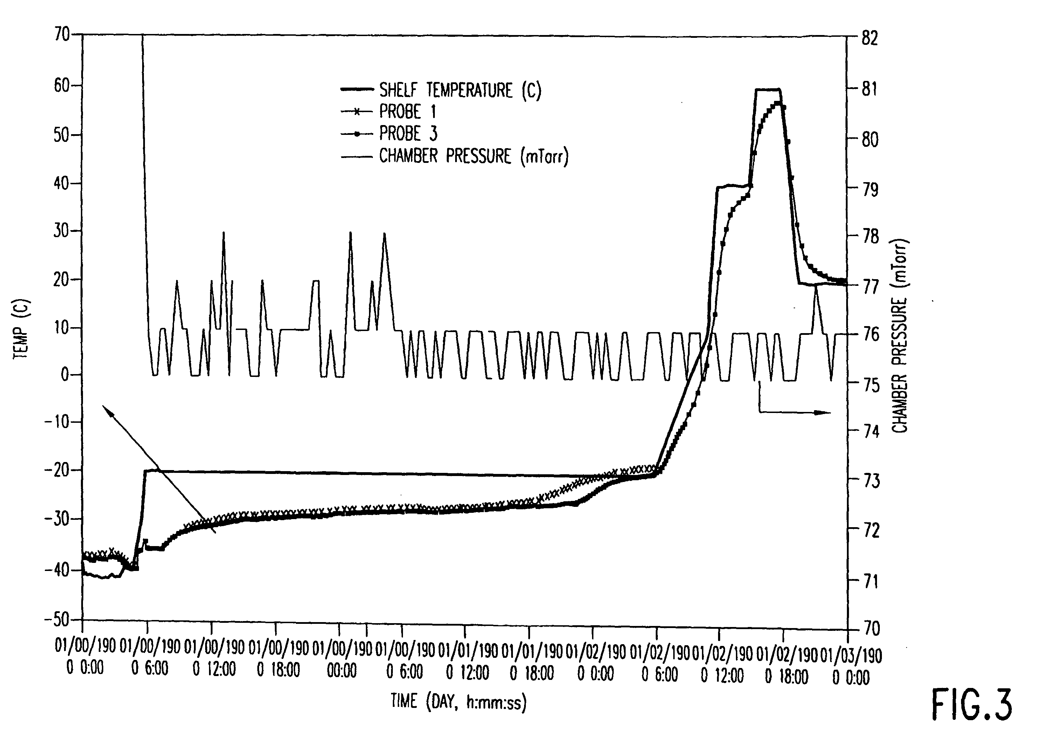

FIG. 3 illustrates the shelf temperature and chamber pressure values during the lyophilization cycle for Examples 1 and 2.

Finally, the vials were removed from the lyophilizer as the final formulation. Upon analysis, the formulation exhibited the following characteristics listed in Table 1:

**Table 1**

| Component | g/liter | g/0.8liter |
|---|---|---|
| Carbapenem | 200.0* | 160.0* |
| Sodium Bicarbonate USP | 35.0 | 28.0 |
| Sodium Hydroxide NF** | adjusted to pH 7.8 | adjusted to pH 7.8 |
| Water for Injection USP*** | q.s 1.00liter | q.s. 0.8liter |

| | | |
|---|---|---|
| * as free acid; | | |
| ** diluted in Water for Injection USP, and used as 2N solution for pH control; | | |
| *** removed during lyophilization | | |

The final product exhibited a moisture content of 1.91 %w/w. Table 2 illustrates the High Performance Liquid Chromatography (HPLC) results in area % of in process samples collected during the production of stabilized carbapenem antibiotic for this example.

**Table 2**

| | Carbapenem | Total Degradates | Total Dimers | Ring Open |
|---|---|---|---|---|
| | HPLC, Area % | | | |
| Bulk drug | 98.6 | 1.4 | 0.5 | 0.7 |
| Prefilter Soln. | 97.6 | 2.3 | 1.1 | 1.0 |
| End of Vial Filling | 96.8 | 3.0 | 1.5 | 1.4 |
| Lyophilized Product | 95.6 | 4.4 | 1.6 | 2.5 |

### Example 2

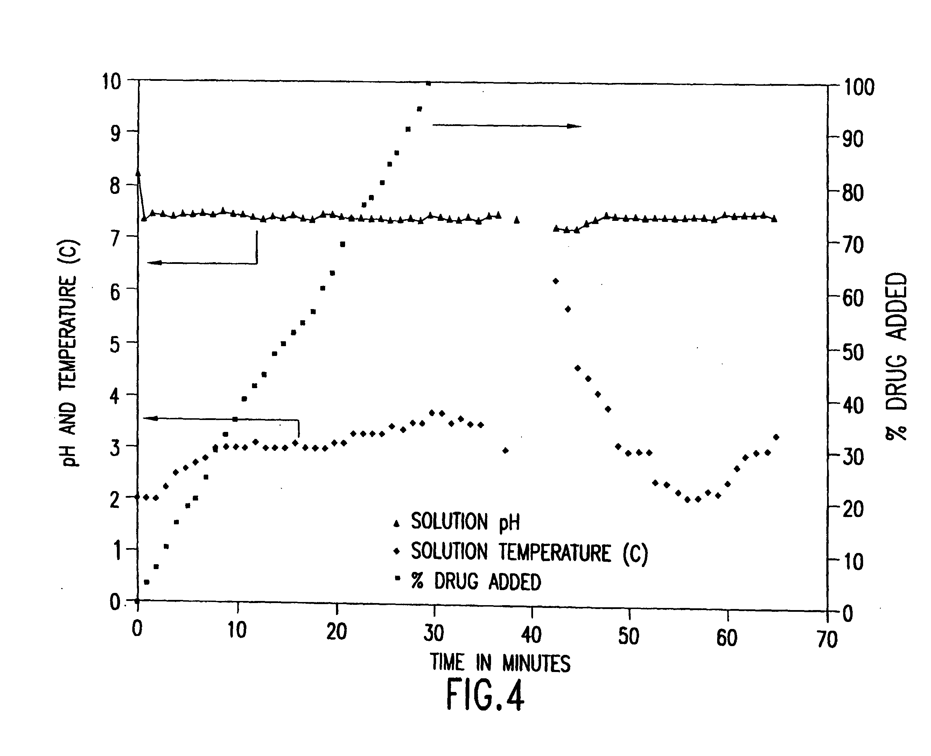

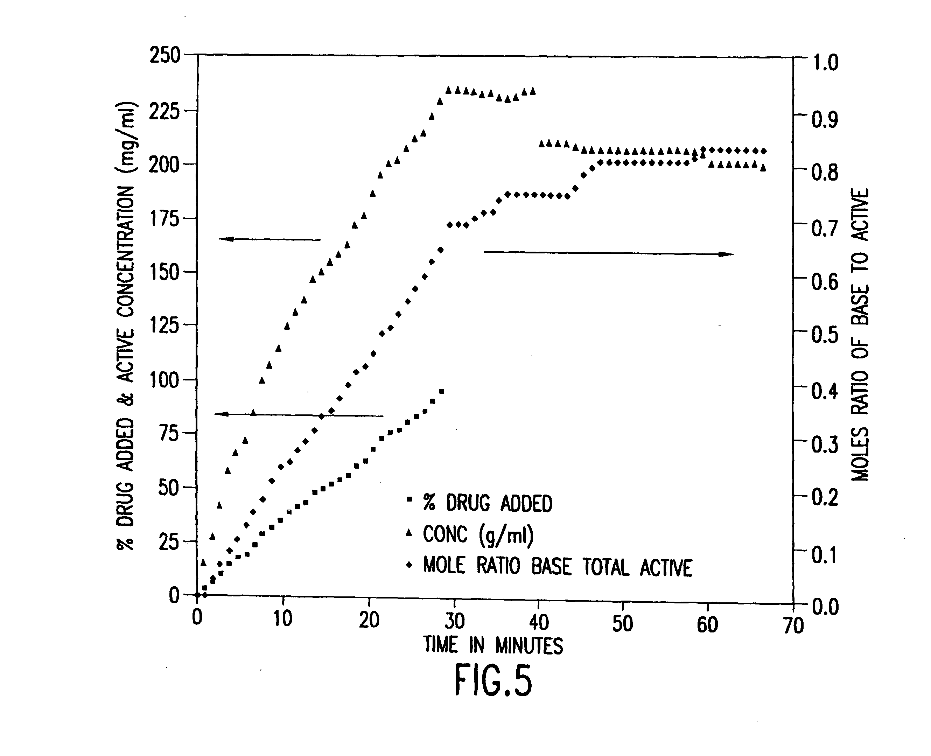

The general procedure described in Example 1 was utilized to prepare the formulation of this example. FIG. 4 illustrates the pH and temperature fluctuations during the total compounding time, and % weight of bulk drug, bulk drug added to the compounder during the compounding period. Except for the values provided in Table 3, identical conditions were utilized in both examples. FIG. 5 provides data for the mole ratio of base to active bulk drug, as well as % bulk drug added to the compounder and active drug concentration during the compounding time. The final product exhibited a moisture content of 1.87 %w/w. Table 4 illustrates the HPLC results in area % of in process samples collected during the production of stabilized carbapenem antibiotic for this example.

**Table 3**

| | |
|---|---|
| Drug Addition Time, minutes | 30 |
| Total Compounding Time, minutes | 68 |
| pH SetPoint during Compounding | 7.4 |
| Mole Ratio of NaOH/Drug | 0.83 |

**Table 4**

| | Carbapenem | Total Degradates | Total Dimers | Ring Open |
|---|---|---|---|---|
| | HPLC, Area % | | | |
| Carbapenem | 98.5 | 1.5 | 0.7 | 0.7 |
| Prefilter Soln. | 98 | 1.9 | 0.9 | 0.9 |
| End of Fill | 97.3 | 2.5 | 1.2 | 1.2 |
| Lyophil' Prod. | 95.9 | 4.1 | 1.5 | 2.3 |

### Examples 3 and 4

Examples 3 and 4 were conducted utilizing the same basic procedures described herein below, with the exception of the parameters given in Table 5, below. However, the vial utilized in Example 3 were conventional, while those utilized in Example 4 were ADD-Vantage™ vials.

To prepare a pilot plant batch of the formulation, a 2N solution of sodium hydroxide was prepared by dissolving 250g of sodium hydroxide NF pellets in 2,000g of WFI while mixing, the solution was cooled to ambient temperature, and WFI was added to produce the final solution of 3,406g. The sodium hydroxide solution was chilled utilizing an Isotemp 1028S Chiller to a temperature of 4°C. Into a 20liter, stainless steel, jacketed compounder, 6.42kg of the WFI was charged, and the solution was cooled to a target temperature of 1° to 5°C. The pH probe attached to a HD-PH-P pH Controller was standardized using pH 7.0 and 10.0 buffer solutions.

Sodium bicarbonate USP in an amount of 448g was dissolved in the compounder while continuously stirring until complete dissolution occurred, wherein the pH of the solution measured 8.3. Thereafter, the unstabilized bulk drug (as anhydrous free acid) in an amount of 2560g was thawed from -20°C to ambient temperature for approximately 1 hour and then divided into 10 equal portions. The 10 portions of bulk drug were added to the compounder over a 60 minute period, while adding the sodium hydroxide solution via the pH controller to keep bulk drug solution in the compounder close to the a target pH of 7.6. At the end of the bulk drug addition, the solution was mixed for an additional 15 minutes, and 2N NaOH titrations were preformed to confirm complete dissolution of the bulk drug. After mixing for an another 15 minutes, water for injection at a temperature of 0° to 8°C was added to bring the solution to about 97% of the total weight, based on 100 total weight percent. While still mixing the solution, the pH thereof was adjusted to 7.7 by addition of 2N NaOH solution, ensuring that the mole ration of NaOH to bulk drug is within the range of 0.8 to 0.9. The weight of the solution was adjusted to 100 weight percent of the final batch weight by addition of WFI at a temperature of from 0° to 8°C while mixing for another 5 minutes. The compounder was then sealed and pressurized to 15psig to initiate filtration, and the solution was filtered through a Millipak 0.22µm sterilizing filter into a sterile receiving vessel, continuously cooled to a temperature for from 1° to 5°C. The filtered, formulation solution exhibited a density of about 1.11 g/ml at 5°C.

The sterile formulation was placed into sterile glass vials (6.33g into 20ml conventional vials, and 5.77g into 15 ml ADD-Vantage). The filled vials were partially stoppered with dry, sterile, siliconized lyophilization stoppers, and placed onto lyophilizer shelves pre-cooled to a temperature of from -45° to -40°C. The lyophilizing procedure was conducted as follows:

### 20 ml Conventional Vials

a. soak at -40°C (range -45° to -40°C) lyo shelf temperature for at least 2 hours;
b. heat to -20°C shelf temperature at 0.5°C/minute;
c. hold shelf temperature at -20°C and 80mTorr pressure for 48 hours;
d. heat to 10°C shelf temperature at 0.1°C/minute;
e. heat to 40°C shelf temperature at 0.5°C/minute; hold at 40°C and 80mTorr for 3 hours;
f. heat to 60°C shelf temperature at 0.5°C/minute; hold at 60°C and 80mTorr for 3 hours;
g. cool the shelves to ambient temperature (20°-30°C) before unloading; and
h. stopper under partial vacuum (target of 0.9 bar/700Torr).

### ADD-Vantage Vials

a. soak at -40°C (range -45° to -40°C) lyophilizer shelf temperature for at least 2 hours;
b. heat to -20°C shelf temperature at 0.5°C/minute;
c. hold shelf temperature at -20°C and 80mTorr pressure for 54 hours;
d. heat to -10°C shelf temperature at 0.1°C/minute; hold at -10°C and 80mTorr for 4 hours;
e. heat to 10°C shelf temperature at 0.1°C/minute;
f. heat to 40°C shelf temperature at 0.5°C/minute; hold at 40°C and 80mTorr for 3 hours;
g. heat to 60°C shelf temperature at 0.5°C/minute; hold at 60°C and 80mTorr for 3 hours;
h. cool the shelves to ambient temperature (20°-30°C) before unloading; and
i. stopper under partial vacuum (target of 0.9 bar/700Torr).

After completion of the lyophilizing step, the vials containing the formulation were removed from the lyophilizer and capped (flip-off caps for conventional vials and ADD-Vantage caps for ADD-Vantage vials). Finally, the vials were stored at a temperature of 25°C or cooler.

**Table 5**

| Image | Example 3 | Example 4 |
|---|---|---|
| Drug Addition Time, min | 45 | 66 |
| Total Compounding Time, min | 114 | 134 |
| pH Controller Setpoint During Drug Addition | 7.6 | 7.6 |
| pH Controller Setpoint During pH Adjustment | 7.7 | 7.7 |
| Mole Ratio of NaOH Added to Active Drug | 0.85** | 0.87** |
| Filtration Time, min | 30 | 31 |
| Vial Filling Time, min | 203 | 157 |
| Lyophilizer Cycle Time, min | 65 | 78 |

The final stabilized carbapenem antibiotic formulation was analyzed to contain the amount of components listed in Table 6, below

**Table 6**

| Component | g/liter | g/0.8liter |
|---|---|---|
| Carbapenem | 200.0* | 160.0* |
| Sodium Bicarbonate USP | 35.0 | 28.0 |
| Sodium Hydroxide NF** | adjusted to pH 7.8 | adjusted to pH 7.8 |
| WFI USP*** | q.s. 1.00liter | q.s. 0.8liter |

Table 7 summarizes the HPLC results of area percent of in-processing samples collected during production of the batch of Example 3.

**Table 7**

| | Carbapenem | Total Degradates | Total Dimers | Ring Opening |
|---|---|---|---|---|
| | HPLC, Area % | | | |
| Bulk Carbapenem | 99.2 | 0.7 | 0.4 | 0.3 |
| Pre-filtered Solution | 97.6 | 2.2 | 1.0 | 1.2 |
| Beginning of Vial Filling | 96.9 | 3.0 | 1.6 | 1.4 |
| Middle of Vial Filling | 96.3 | 3.0 | 1.6 | 1.4 |
| End of Vial Filling | 95.7 | 4.3 | 2.5 | 1.7 |
| Beginning of Lyophilization | 95.5 | 4.4 | 1.7 | 2.5 |
| Middle of Lyophilization | 95.2 | 4.6 | 1.9 | 2.5 |
| End of Lyophilization | 94.7 | 5.2 | 2.3 | 2.7 |
| The weight percent of moisture per 100% total weight, as determined by NIR for Examples 3 and 4 were 1.8 and 2.1, respectively. | | | | |

The principles of the process and formulations, preferred embodiment and modes of operation of the present invention have been described in the foregoing specification. However, the invention disclosed as intended to be protected is not to be construed as limited to the particular embodiments disclosed. The embodiments are to be construed as illustrative rather than restrictive. It is recognized, however, that departures may be made therefrom within the scope of the invention, and that obvious modifications may occur to a person skilled in the art.

## Claims

1. A process for converting an unstablized, monosodium adduct of carbapenem of formula I into a stabilized, carbon dioxide adduct of carbapenem for the treatment of bacterial infections in mammal patients: 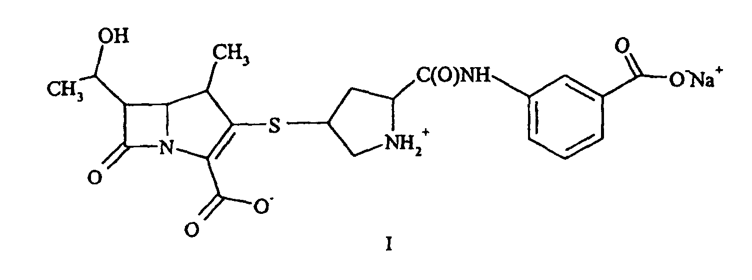 comprising the steps of:
a. preparing a solution of 1 to 3N sodium hydroxide, chilling the solution to a temperature of from 0°C to 10°C;
b. charging from 40 to 60% by wt., based on 100% by wt. total batch weight, of Water for Injection into a compounder having means for mixing, and cooling the water to a temperature of from 0°C to 10°C;
c. charging 1 mole equivalent of carbonate/active carbapenem, wherein the carbonate is selected from sodium bicarbonate, sodium carbonate and mixtures thereof, into the compounder while mixing, to prepare a carbonate solution, while maintaining a temperature of from 0°C to 10°C;
d. maintaining the carbonate solution at a temperature range of from about 0°C to about 10°C, and a pH of from 7.5 to 9.0;
e. thawing a sufficient amount of the unstabilized carbapenem from a temperature of -20°C to a temperature of from 5°C to 25°C to prepare a final formulation containing about 200 g/liter of the active carbapenem, and charging at the same time into the compounder from 0.7 to 1.0 mole of sodium hydroxide/mole of the active carbapenem, while mixing the carbonate solution to dissolve the carbapenem therein, and maintaining the compounder temperature of from 0° to 5°C to produce a beta-lactam-carbonate solution;
f. adding the sodium hydroxide solution to the carbapenem-carbonate solution, as required, during step e. to maintain the pH of the solution of from 7.0 to 8.0;
g. adding water, as required, to adjust the carbapenem-carbonate solution to a range of 95 to 97 weight %, based on 100 total weight %, and maintaining a temperature of from 0° to 5°C;
h. adding the sodium hydroxide solution to the carbapenem-carbonate solution, as required, to maintain the solution in a pH of from 7.2 to 7.8;
i. adding water, as required, to adjust the carbapenem-carbonate solution to 100 weight % total, and maintaining the temperature of from 0° to 5°C;
j. sealing the compounder containing the carbapenem-carbonate solution and pressurizing to from 0.7 bar (10 psig) to 2.1 bar (30 psig) to initialize filtration;
k. filtering the carbapenem-carbonate solution through a sterilizing filter into a continuously cooled, sterile, receiving vessel exhibiting a temperature of from 0° to 5°C to produce a sterile, stabilized carbapenem formulation;
l. aseptically filling the formulation into sterilized glass vials;
m. partially sealing the glass vials with dry, sterilized stoppers;
n. lyophilizing the solution by freezing in the glass vials at a temperature of from -45° to 40°C to produce a frozen formulation;
o. primary drying the frozen formulation at a temperature of from about -25 to -15°C for 48 to 60 hours at a pressure of about 0.1 mbar (80mTorr) or lower;
p. secondary drying the formulation at a temperature from 40° to 60°C at pressure of about 0.1 mbar (80mTorr) or lower for from 3 to 10 hours;
q. cooling the vials to ambient temperature; and
r. sealing the vials under a partial vacuum, while maintaining a temperature of about 25°C.

2. The process according to Claim 1, wherein the carbon dioxide adduct of carbapenem is of formula II:

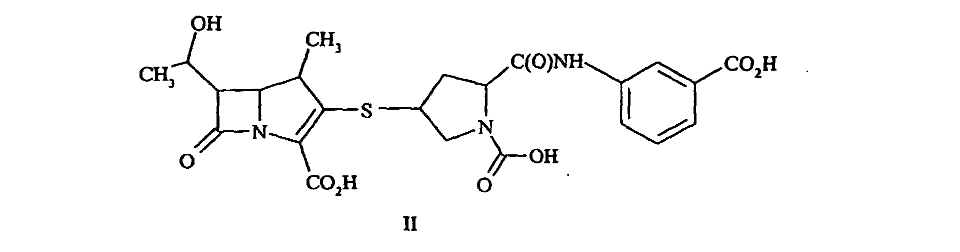

3. The process according to Claim 1, wherein the concentration of sodium bicarbonate to water in the solution is about 35g/liter.

4. The process according to Claim 1, wherein the pH of sodium bicarbonate solution is about 8.25.

5. The process according to Claim 1, wherein the bulk compound of formula I exhibits a moisture content of up to 20.0% by weight.

6. The process according to Claim 3, wherein the sodium hydroxide solution is high velocity injected into the compounder.

7. The process according to Claim 4, wherein the reaction vessel is pressurized to a pressure of about 1 bar (15 psig).

8. The process according to Claim 5, wherein the sterilizing microfilter is about 0.22µm.

9. The process according to Claim 6, wherein the lyophilizing step, comprises:
a. cooling the vials to a temperature of from -45° to -40°C for about 2 hours;
b. heating the vials to a temperature of about -20°C at a rate of about 0.5°C/minute, and maintaining the vial temperature at about -20°C, while maintaining a pressure of from 0.9 mbar (65mTorr) to 0.13 mbar (95mTorr) for about 48 hours;
c. heating the vials to a temperature of about 10°C at a rate of about 0.1°C/min;
d. heating the vials to a temperature of about 40°C at a rate of about 0.5°C/min, and hold at about 40°C, while maintaining a pressure of about 0.1 mbar (80mTorr) or lower for up to about 3 hours;
e. heating the vials to a temperature of about 60°C at a rate of about 0.5°C/min, and hold at about 60°C and about 0.1 mbar (80mTorr) or lower for about 5 hours; and
f. cooling the vials to a temperature of from 20° to 30°C.

10. The process according to Claim 7, wherein the vials are sealed under a partial vacuum of about 0.9 bar (700Torr) or lower.

11. The process according to Claim 8, wherein the stabilized carbapenem of formula II exhibits a carbapenem concentration of 200 g/liter, and a sodium bicarbonate concentration of 35.0g/liter.

12. A process for converting an unstabilized, carbapenem compound of formula I: 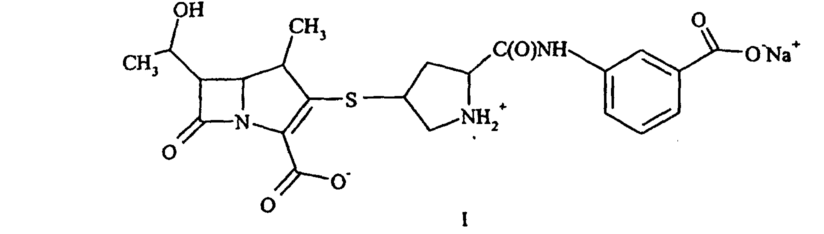 into a stabilized, carbapenem compound of formula II: 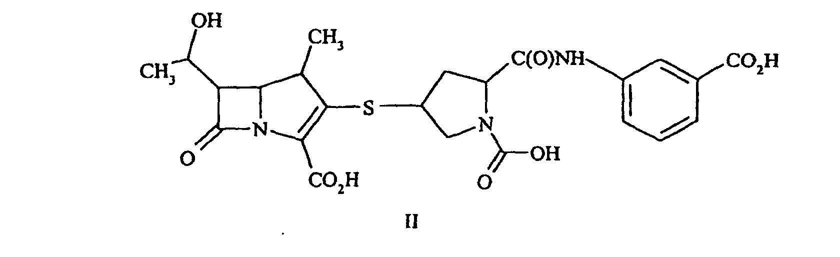 comprising the steps of:
a. preparing a solution of from about 1 to about 3N of sodium hydroxide and chilling the solution to a temperature of from 0° to 10°C;
b. charging a total of from 40 to 60% by wt., based on 100% by wt. total of the batch weight of Water for Injection exhibiting a temperature of from 2° to 85°C into a compounder having means for mixing, and cooling the water to a temperature of from 0° to 5°C;
c. charging sufficient carbonate selected from sodium bicarbonate, sodium carbonate and mixtures thereof, into the compounder to prepare a final formulation exhibiting 1 mole equivalent of carbonate/active carbapenem, dissolving the same, and maintaining the solution at a temperature range of from 0° to 5°C to prepare a carbonate solution;
d. maintaining the carbonate solution at a pH of from 7.5 to 9.0, and a temperature of 0° to 5°C;
e. thawing a sufficient amount of bulk carbapenem of formula I to provide a formulation exhibiting a concentration of about 200 g/l from a temperature of -20°C to temperature of from 5 to 25°C, and slowly charging the same into the compounder with mixing of the carbonate solution to completely dissolve the bulk compound, while maintaining the compounder temperature of from 0° to 5°C to produce an active carbapenem solution;
f. adding the sodium hydroxide solution to the active carbapenem, as required during step e., and maintaining a pH of from 7.0 to 8.0;
g. adjusting the active carbapenem solution to 95% by weight of the final product weight, based on 100 total weight percent, utilizing water for injection, as required and maintaining the bulk carbapenem solution in a temperature of from 0° to 5°C;
h. adding the sodium hydroxide solution to the bulk carbapenem solution to maintain a pH of from 7.2 to 7.8;
i. adding Water for Injection, as required, to adjust the active carbapenem solution to q.s of 100% by wt., based 100% by wt. total weight, and maintaining a temperature of from about 0 to 5°C;
j. sealing and pressurizing the compounder containing the bulk compound solution to about I bar (15 psig) to initiate filtration:
k. filtering the bulk compound solution through a sterilizing filter into a continuously cooled, sterile, receiving vessel exhibiting a temperature of from 0° to 5°C; and
l. aseptically filling the intravenous formulation into sterile glass vials, and partially sealing the vials with dry, sterilized, lyophilization stoppers;
m. lyophilizing the formulation by freezing in the glass vials at a temperature of from -45° to -40°C to produce a frozen formulation;
n. primary drying the frozen formulation at a temperature of from -25° to -15°C for 48 to 60 hours at a pressure of about 0.1mbar (80mTorr);
o. secondary drying the formulation at a temperature of from 40° to 60°C at a pressure of about 0.1 mbar (80mTorr) or lower for a time period of from 3 to 10 hours;
p. cooling the vials to ambient temperature; and
q. sealing the veals under a partial vacuum of 0.9 bar (700Torr) or lower, while maintaining a temperature of about 25°C,
wherein the stabilized, carbapenem antibiotic formulation of formula II exhibits a carbapenem concentration of about 200g/l and a carbonate content of about 1 mole equivalent.

13. The process according to Claim 12, wherein the bulk compound to sodium hydroxide solution concentration is 1 mole of bulk compound/0.85 moles of sodium hydroxide.

14. The process according to Claim 13, wherein the lyophilized solution exhibits a density of about 1.11 g/ml.

15. The process according to Claim 14, wherein in step j. the temperature in the compounder is about 2°C.

16. The process according to Claim 15, wherein in step e. the temperature when dissolving the bulk drug into the sodium bicarbonate solution is 2°C.

17. The process according to Claim 16, wherein in step l the temperature when filling the formulation into vials is from 0° to 5°C.

18. The process according to Claim 17, wherein the step o. lyophilized, sterile formulation exhibits a density of about 1.11g/ml at 5°C

19. The process according to Claim 18, wherein the stabilized carbapenem formulation exhibits a concentration of about 200g of carbapenem of formula II per liter of total solution.

## Patentansprüche

1. Ein Verfahren zur Umwandlung eines nichtstabilisierten Mononatriumaddukts des Carbapenems der Formel I in ein stabilisiertes Kohlendioxidaddukt des Carbapenems zur Behandlung von Bakterieninfektionen bei Säugetierpatienten: 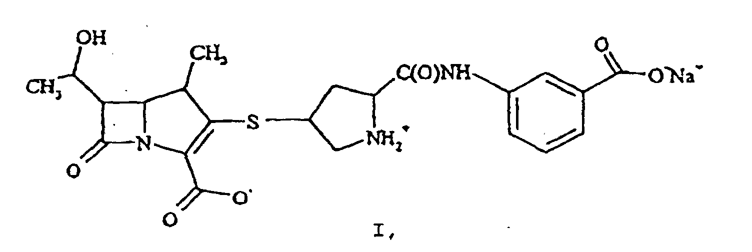 umfassend die Schritte:
a. Herstellen einer 1N bis 3N Natriumhydroxidlösung, Abkühlen der Lösung auf eine Temperatur von 0°C bis 10°C,
b. Einbringen von 40 bis 60 Gew.-%, bezogen auf 100 Gew.-% Chargengesamtgewicht, Wasser zur Injektion in einen Compounder mit Mitteln zum Vermischen und Abkühlen des Wassers auf eine Temperatur von 0°C bis 10°C,
c. Einbringen von 1 Moläquivalent Carbonat/aktivem Carbapenem, wobei das Carbonat ausgewählt ist aus Natriumhydrogencarbonat, Natriumcarbonat und Mischungen davon, in den Compounder unter Vermischen, um eine Carbonatlösung herzustellen, während eine Temperatur von 0°C bis 10°C aufrechterhalten wird,
d. Halten der Carbonatlösung bei einer Temperatur im Bereich von etwa 0°C bis etwa 10°C und einem pH-Wert von 7,5 bis 9,0,
e. Auftauen einer ausreichenden Menge des nichtscabilisierten Carbapenems von einer Temperatur von -20°C auf eine Temperatur von 5°C bis 25°C, um eine Endformulierung herzustellen, die etwa 200 g/Liter des aktiven Carbapenems enthält, und gleichzeitig Einbringen von 0,7 bis 1,0 Mol Natriumhydroxid/Mol des aktiven Carbapenems in den Compounder, während die Carbonatlösung gemischt wird, um das darin befindliche Carbapenem aufzulösen, und Aufrechterhalten einer Compoundertemperatur von 0°C bis 5°C, um eine Betalactam-Carbonat-Lösung zu erzeugen,
f. Zugeben der Natriumhydroxidlösung zu der Carbapenem-Carbonat-Lösung während Schritt e. nach Bedarf, um den pH-Wert der Lösung bei 7,0 bis 8,0 zu halten,
g. Zugeben von Wasser nach Bedarf, um die Carbapenem-Carbonat-Lösung auf einen Bereich von 95 bis 97 Gew.-%, bezogen auf insgesamt 100 Gew.-%, einzustellen, und Aufrechterhalten einer Temperatur von 0°C bis 5°C,
h. Zugeben der Natriumhydroxidlösung zur Carbapenem-Carbonat-Lösung nach Bedarf, um die Lösung bei einem pH-Wert von 7,2 bis 7,8 zu halten,
i. Zugeben von Wasser nach Bedarf, um die Carbapenem-Carbonat-Lösung auf insgesamt 100 Gew.-% einzustellen, und Aufrechterhalten der Temperatur von 0°C bis 5°C,
j. Verschließen des Compounders, der die Carbapenem-Carbonac-Lösung enthält, und Aufbauen eines Drucks von 0,7 bar (10 psig) bis 2,1 bar (30 psig), um die Filtration zu initiieren,
k. Filtrieren der Carbapenem-Carbonat-Lösung durch ein Sterilisationsfilter in ein fortlaufend gekühltes steriles Auffanggefäß, das eine Temperatur von 0°C bis 5°C aufweist, um eine sterile stabilisierte Carbapenem-Formulierung zu erzeugen,
l. aseptisches Einfüllen der Formulierung in sterilisierte Glasampullen,
m. teilweises Verschließen der Glasampullen mit trockenen sterilisierten Stopfen,
n. Gefriertrocknen der Lösung durch Einfrieren der Glasampullen bei einer Temperatur von -45°C bis -40°C, um eine gefrorene Formulierung zu erzeugen,
o. anfängliches Trocknen der gefrorenen Formulierung bei einer Temperatur von -25°C bis -15°C 48 bis 60 Stunden lang bei einem Druck von etwa 0,1 mbar (80 mTorr) oder weniger,
p. nachfolgendes Trocknen der Formulierung bei einer Temperatur von 40'C bis 60°C 3 bis 10 Stunden lang bei einem Druck von etwa 0,1 mbar (80 mTorr) oder weniger,
q. Abkühlen der Ampullen auf Umgebungstemperatur und
r. Verschließen der Ampullen unter Teilvakuum, während eine Temperatur von etwa 25°C aufrechterhalten wird.

2. Das Verfahren gemäß Anspruch 1, wobei das Kohlendioxid-Addukt des Carbapenems die Formel II besitzt:

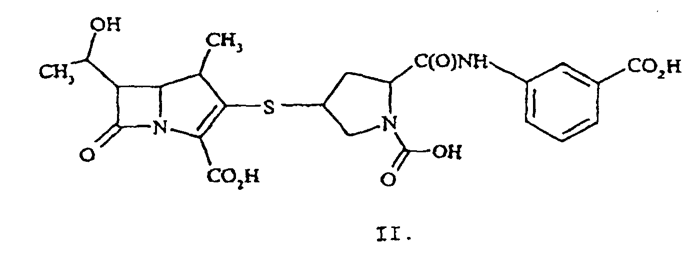

3. Das Verfahren gemäß Anspruch 1, wobei die Konzentration von Natriumhydrogencarbonat zu Wasser in der Lösung etwa 35 g/Liter beträgt.

4. Das Verfahren gemäß Anspruch 1, wobei der pH-Wert der Natriumhydrogencarbonatlösung etwa 8,25 beträgt.

5. Das Verfahren gemäß Anspruch 1, wobei die Rohverbindung der Formel I einen Feuchtigkeitsgehalt von bis zu 20,0 Gew.-% aufweist.

6. Das Verfahren gemäß Anspruch 3, wobei die Natriumhydroxidlösung mit hoher Geschwindigkeit in den Compounder eingespritzt wird.

7. Das Verfahren gemäß Anspruch 4, wobei im Reaktionsgefäß ein Druck von etwa 1 bar (15 psig) aufgebaut wird.

8. Das Verfahren gemäß Anspruch 5, wobei das Sterilisations-Mikrofilter etwa 0,22 µm ist.

9. Das Verfahren gemäß Anspruch 6, wobei der Gefriertrocknungsschritt umfaßt:
a. Abkühlen der Ampullen auf eine Temperatur von -45°C bis -40°C etwa 2 Stunden lang,
b. Erwärmen der Ampullen auf eine Temperatur von etwa -20°C mit einer Geschwindigkeit von etwa 0,5°C/Minute und Aufrechterhalten der Ampullentemperatur bei etwa -20°C etwa 48 Stunden lang, während ein Druck von 0,9 mbar (65 mTorr) bis 0,13 mbar (95 mTorr) aufrechterhalten wird,
c. Erwärmen der Ampullen auf eine Temperatur von etwa 10°C mit einer Geschwindigkeit von etwa 0,1°C/Minute,
d. Erwärmen der Ampullen auf eine Temperatur von etwa 40°C mit einer Geschwindigkeit von etwa 0,5°C/Minute und Aufrechterhalten der Temperatur bei etwa 40°C bis zu etwa 3 Stunden lang, während ein Druck von etwa 0,1 mbar (80 mTorr) oder weniger aufrechterhalten wird,
e. Erwärmen der Ampullen auf eine Temperatur von etwa 60°C mit einer Geschwindigkeit von etwa 0,5°C/Minute und Aufrechterhalten der Temperatur bei etwa 60°C und einem Druck von etwa 0,1 mbar (80 mTorr) oder weniger etwa 5 Stunden lang und
f. Abkühlen der Ampullen auf eine Temperatur von 20°C bis 30°C.

10. Das Verfahren gemäß Anspruch 7, wobei die Ampullen unter einem Teilvakuum von etwa 0,9 bar (700 Torr) oder weniger verschlossen werden.

11. Das Verfahren gemäß Anspruch 8, wobei das stabilisierte Carbapenem der Formel II eine Carbapenemkonzentration von 200 g/Liter und eine Natriumhydrogencarbonatkonzentration von 35,0 g/Liter aufweist.

12. Ein Verfahren zur Umwandlung einer nichtstabilisierten Carbapenem-Verbindung der Formel I: 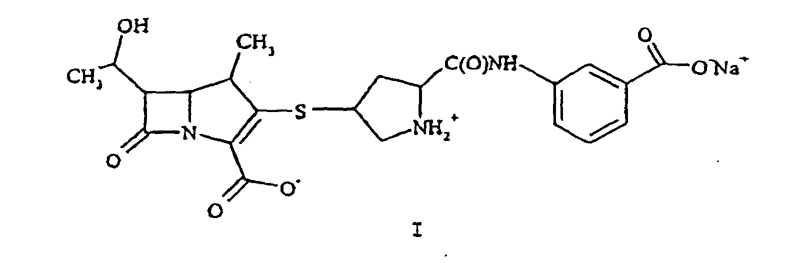 in eine stabilisierte Carbapenem-Verbindung der Formel II: 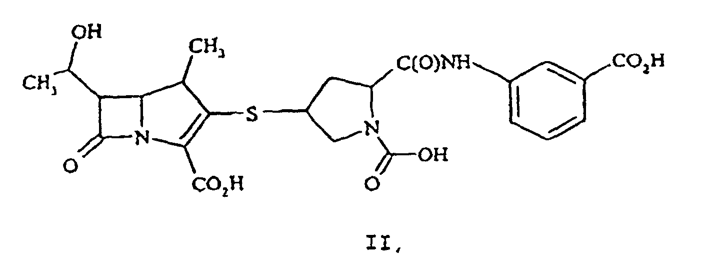 umfassend die Schritte:
a. Herstellen einer etwa 1N bis etwa 3N Natriumhydroxidlösung und Abkühlen der Lösung auf eine Temperatur von 0°C bis 10°C,
b. Einbringen von insgesamt 40 bis 60 Gew.-%, bezogen auf 100 Gew.-% Chargengesamtgewicht, Wasser zur Injektion mit einer Temperatur von 2°C bis 85°C in einen Compounder mit Mitteln zum Vermischen und Abkühlen des Wassers auf eine Temperatur von 0°C bis 5°C,
c. Einbringen von ausreichend Carbonat, ausgewählt aus Natriumhydrogencarbonat, Natriumcarbonat und Mischungen davon, in den Compounder, um eine Endformulierung herzustellen, die 1 Moläquivalent Carbonat/aktives Carbapenem aufweist, Auflösen desselben und Halten der Lösung bei einer Temperatur im Bereich von 0°C bis 5°C, um eine Carbonatlösung herzustellen,
d. Halten der Carbonatlösung bei einem pH-Wert von 7,5 bis 9,0 und einer Temperatur von 0°C bis 5°C,
e. Auftauen einer ausreichenden Menge des Rohcarbapenems der Formel I, um eine Formulierung bereitzustellen, die eine Konzentration von etwa 200 g/l aufweist, von einer Temperatur von -20°C auf eine Temperatur von 5°C bis 25°C und langsames Einbringen derselben in den Compounder, während die Carbonatlösung gemischt wird, um die Rohverbindung vollständig aufzulösen, wobei die Compoundertemperatur bei 0°C bis 5°C gehalten wird, um eine aktive Carbapenemlösung zu erzeugen,
f. Zugeben der Natriumhydroxidlösung zu dem aktiven Carbapenem während Schritt e. nach Bedarf und Aufrechterhalten eines pH-Werts von 7,0 bis 8,0,
g. Einstellen der Lösung des aktiven Carbapenems auf 95 Gew.-% des Endproduktgewichts, bezogen auf insgesamt 100 Gew.-%, wobei Wasser zur Injektion nach Bedarf verwendet wird, und Halten der Rohcarbapenemlösung bei einer Temperatur von 0°C bis 5°C,
h. Zugeben der Natriumhydroxidlösung zur Rohcarbapenemlösung, um einen pH-Wert von 7,2 bis 7,8 aufrechtzuerhalten,
i. Zugeben von Wasser zur Injektion nach Bedarf, um die Lösung des aktiven Carbapenems auf q.s. 100 Gew.-% einzustellen, bezogen auf 100 Gew.-% Gesamtgewicht, und Aufrechterhalten einer Temperatur von 0°C bis 5°C,
j. Verschließen des Compounders, der die Rohverbindungslösung enthält, und Aufbauen eines Drucks von etwa 1 bar (15 psig), um die Filtration zu initiieren,
k. Filtrieren der Rohvexbindungelösung durch ein Sterilisationsfilter in ein fortlaufend gekühltes steriles Auffanggefäß, das eine Temperatur von 0°C bis 5°C aufweist, und
l. aseptisches Einfüllen der intravenösen Formulierung in sterile Glasampullen und teilweises Verschließen der Ampullen mit trockenen sterilisierten Gefriertrocknungsstopfen,
m. Gefriertrocknen der Formulierung durch Einfrieren in den Glasampullen bei einer Temperatur von -45°C bis -40°C, um eine gefrorene Formulierung zu erzeugen,
n. anfängliches Trocknen der gefrorenen Formulierung bei einer Temperatur von -25°C bis -15°C 48 bis 60 Stunden lang bei einem Druck von etwa 0,1 mbar (80 mTorr),
o. nachfolgendes Trocknen der Formulierung bei einer Temperatur von 40°C bis 60°C für einen Zeitraum von 3 bis 10 Stunden bei einem Druck von etwa 0,1 mbar (80 mTorr) oder weniger,
p. Abkühlen der Ampullen auf Umgebungstemperatur und
q. Verschließen der Ampullen unter einem Teilvakuum von 0,9 bar (700 Torr) oder weniger, während eine Temperatur von etwa 25°C aufrechterhalten wird,
wobei die stabilisierte antibiotische Carbapenem-Formulierung der Formel II eine Carbapenem-Konzentration von etwa 200 g/l und einen Carbonatgehalt von etwa 1 Moläquivalent aufweist.

13. Das Verfahren gemäß Anspruch 12, wobei die Konzentration der Rohverbindung zur Natriumhydroxidlösung 1 Mol Rohverbindung/0,85 Mol Natriumhydroxid beträgt.

14. Das Verfahren gemäß Anspruch 13, wobei die gefriergetrocknete Lösung eine Dichte von etwa 1,11 g/ml aufweist.

15. Das Verfahren gemäß Anspruch 14, wobei in Schritt j. die Temperatur im Compounder etwa 2°C beträgt.

16. Das Verfahren gemäß Anspruch 15, wobei in Schritt e. die Temperatur beim Auflösen des Roharzneistoffes in der Natriumhydrogencarbonatlösung 2°C beträgt.

17. Das Verfahren gemäß Anspruch 16, wobei in Schritt 1. die Temperatur beim Einfüllen der Formulierung in Ampullen 0°C bis 5°C beträgt.

18. Das Verfahren gemäß Anspruch 17, wobei die gefriergetrocknete sterile Formulierung von Schritt o. eine Dichte von etwa 1,11 g/ml bei 5°C aufweist.

19. Das Verfahren gemäß Anspruch 18, wobei die stabilisierte Carbapenemformulierung eine Konzentration von etwa 200 g Carbapenem der Formel II pro Liter Gesamtlösung aufweist.

## Revendications

1. Procédé pour convertir un produit d'addition monosodique de carbapénème non stabilisé de formule I en un produit d'addition avec le dioxyde de carbone de carbapénème stabilisé, pour le traitement d'infections bactériennes chez des patients mammifères. 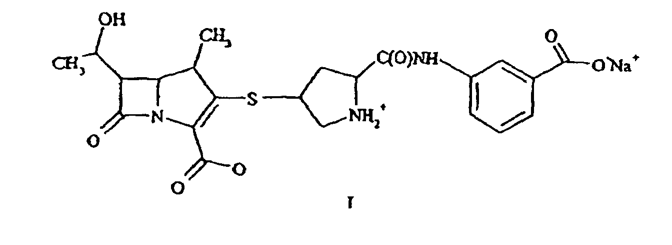 comprenant les étapes consistant à :
a. préparer une solution d'hydroxyde de sodium 1 à 3 N, refroidir la solution à une température de 0 °C à 10 °C ;
b. introduire de 40 à 60 % en poids, par rapport à 100 % en poids du poids total du lot, d'eau pour injection dans un appareil de malaxage doté d'un moyen de mélange et refroidir l'eau à une température de 0 °C à 10 °C;
c. introduire 1 équivalent molaire de carbonate/carbapénème actif, le carbonate étant choisi parmi le bicarbonate de sodium, le carbonate de sodium et leurs mélanges dans l'appareil de malaxage tout en mélangeant, afin de préparer une solution de carbonate en maintenant une température de 0 °C à 10 °C ;
d. maintenir la solution de carbonate dans un intervalle de températures d'environ 0 °C à environ 10 °C, et à un pH de 7,5 à 9,0 ;
e. décongeler une quantité suffisante du carbapénème non stabilisé d'une température de -20 °C à une température de 5 °C à 25 °C, afin de préparer une formulation finale contenant environ 200 g/litre du carbapénème actif, et introduire en même temps dans l'appareil de malaxage de 0,7 à 1,0 mole d'hydroxyde de sodium/mole du carbapénème actif, tout en mélangeant la solution de carbonate afin de dissoudre le carbapénème dedans, et maintenir la température de l'appareil de malaxage de 0 °C à 5 °C afin de produire une solution de bêta-lactame-carbonate ;
f. ajouter la solution d'hydroxyde de sodium à la solution de carbapénème-carbonate, selon les besoins, pendant l'étape e. pour maintenir le pH de la solution de 7,0 à 8,0 ;
g. ajouter de l'eau, selon les besoins, afin d'ajuster la solution de carbapénème-carbonate dans un intervalle de 95 à 97 % en poids, par rapport à 100 % de poids total, et maintenir une température de 0 ° à 5 °C ;
h. ajouter la solution d'hydroxyde de sodium à la solution de carbapénème-carbonate, selon les besoins, pour maintenir la solution à un pH de 7,2 à 7,8 ;
i. ajouter de l'eau, selon les besoins, pour ajuster la solution de carbapénème-carbonate à 100 % du poids total, et maintenir la température de 0 ° à 5 °C ;
j. sceller l'appareil de malaxage contenant la solution de carbapénème-carbonate et à pressuriser de 0,7 bar (10 psig) à 2,1 bars (30 psig) pour initialiser la filtration ;
k. filtrer la solution de carbapénème-carbonate sur un filtre stérilisant dans un récipient récepteur stérile refroidi en continu présentant une température de 0 ° à 5 °C afin de produire une formulation de carbapénème stabilisé stérile ;
l. remplir en conditions aseptiques des flacons de verre stérilisés de la formulation ;
m. sceller partiellement les flacons de verre avec des bouchons secs stérilisés ;
n. lyophiliser la solution par congélation dans les flacons de verre à une température de -45° à -40 °C pour produire une formulation congelée ;
o. effectuer un séchage primaire de la formulation congelée à une température de -25 à -15 °C pendant 48 à 60 heures à une pression d'environ 0,1 mbar (80 mTorr) ou moins ;
p. effectuer un séchage secondaire de la formulation à une température de 40 ° à 60 °C à une pression d'environ 0,1 mbar (80 mTorr) ou moins pendant environ 3 à 10 heures ;
q. refroidir les flacons à température ambiante ; et
r. sceller les flacons sous vide partiel, tout en maintenant une température d'environ 25 °C.

2. Procédé selon la revendication 1, dans lequel le produit d'addition de carbapénème avec du dioxyde de carbone a la formule II :

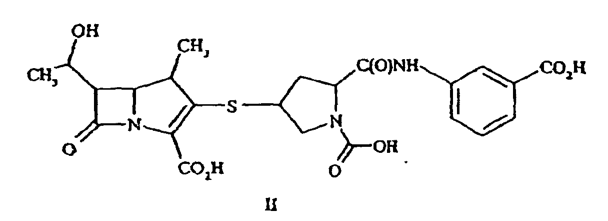

3. Procédé selon la revendication 1, dans lequel la concentration de bicarbonate de sodium par rapport à l'eau dans la solution est d'environ 35 g/litre.

4. Procédé selon la revendication 1, dans lequel le pH de la solution de bicarbonate de sodium est d'environ 8,25.

5. Procédé selon la revendication 1, dans lequel le volume de composé de formule I en vrac présente une teneur en humidité allant jusqu'à 20,0 % en poids.

6. Procédé selon la revendication 3, dans lequel la solution d'hydroxyde de sodium est injectée à haute vitesse dans l'appareil de malaxage.

7. Procédé selon la revendication 4, dans lequel le récipient de réaction est pressurisé à une pression d'environ 1 bar (15 psig).

8. Procédé selon la revendication 5, dans lequel le microfiltre stérilisant est d'environ 0,22 µm.

9. Procédé selon la revendication 6, dans lequel l'étape de lyophilisation comprend :
a. le fait de refroidir les flacons à une température de - 45 °C à -40 °C pendant environ 2 heures ;
b. le fait de chauffer les flacons à une température d'environ -20 °C à une vitesse d'environ 0,5 °C/minutes, et de maintenir la température des flacons à environ -20 °C, tout en maintenant une pression de 0,9 mbar (35 mTorr) à 0,13 mbar (95 mTorr) pendant environ 48 heures ;
c. le fait de chauffer les flacons à une température d'environ 10 °C à une vitesse d'environ 0,1 °C/minute ;
d. le fait de chauffer les flacons à une température d'environ 40 °C à une vitesse d'environ 0,5 °C/min, et de les maintenir à environ 40 °C, tout en maintenant une pression d'environ 0,1 mbar (80 mTorr) ou moins pendant une durée allant jusqu'à 3 heures ;
e. le fait de chauffer les flacons à une température d'environ 60 °C à une vitesse d'environ 0,5 °C/min, et de les maintenir à environ 60 °C et environ 0,1 mbar (80 mTorr) ou moins pendant environ 5 heures ; et
f. le fait de refroidir les flacons à une température de 20 ° à 30 °C.

10. Procédé selon la revendication 7, dans lequel les flacons sont scellés sous un vide partiel d'environ 0,9 bar (700 Torr) ou moins.

11. Procédé selon la revendication 8, dans lequel le carbapénème stabilisé de formule II présente une concentration de carbapénème de 200 g/litre, et une concentration de bicarbonate de sodium de 35,0 g/litre.

12. Procédé pour convertir un composé de carbapénème non stabilisé de formule I : 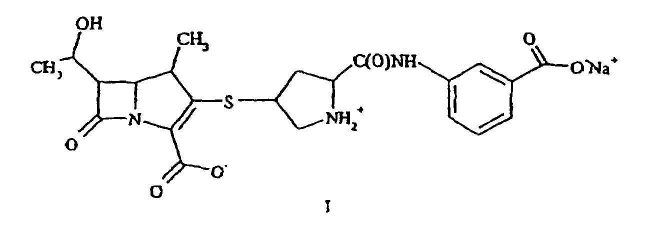 en un composé de carbapénème stabilisé de formule II : 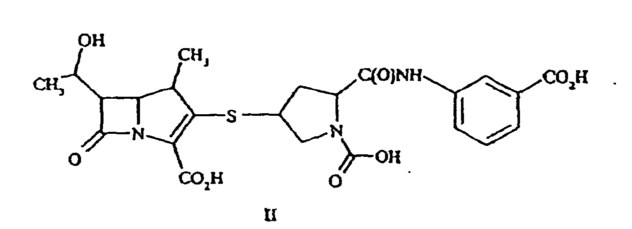 comprenant les étapes consistant à :
a. préparer une solution d'hydroxyde de sodium d'environ 1 environ 3 N et refroidir la solution à une température de 0 ° à 10 °C ;
b. introduire de 40 à 60 % en poids, par rapport à 100 % en poids du poids total du lot, d'eau pour injection présentant une température de 2 ° à 85 °C dans un appareil de malaxage doté d'un moyen de mélange et refroidir l'éau à une température de 0 °C à 5 °C;
c. charger suffisamment de carbonate, choisi parmi du bicarbonate de sodium, du carbonate de sodium et leurs mélanges, dans l'appareil de malaxage pour préparer une formulation finale présentant 1 équivalent molaire de carbonate/carbapénème actif, dissoudre celle-ci, et maintenir la solution dans un intervalle de températures de 0 ° à 5 °C pour préparer une solution de carbonate ;
d. maintenir la solution de carbonate à un pH de 7,5 à 9,0 et à une température, de 0 °C à 5 °C ;
e. décongeler une quantité suffisante du carbapénème en vrac de formule I, pour obtenir une formulation ayant une concentration d'environ 200 g/l, d'une température de -20 °C à une température de 5 °C à 25 °C, et de l'introduire lentement dans l'appareil de malaxage, tout en mélangeant la solution de carbonate afin de dissoudre complètement le composé en vrac, tout en maintenant la température de l'appareil de malaxage de 0 °C à 5 °C afin de produire une solution de carbapénème actif ;
f. ajouter la solution d'hydroxyde de sodium au carbapénème actif, selon les besoins, pendant l'étape e, et maintenir un pH de 7,0 à 8,0 ;
g. ajuster la solution de carbapénème actif à 95 % en poids du poids du produit final, par rapport à 100 % en poids, en utilisant de l'eau pour injection, selon les besoins et maintenir la solution de carbapénème en vrac à une température de 0° à 5 °C ;
h. ajouter la solution d'hydroxyde de sodium à la solution de carbapénème en vrac pour maintenir un pH de 7,2 à 7,8 ;
i. ajouter de l'eau pour injection, selon les besoins, pour ajuster la solution de carbapénème actif à q.s de 100 % en poids, par rapport à 100 % du poids total, et à maintenir une température de 0 à 5 °C ;
j. sceller et pressuriser l'appareil de malaxage contenant la solution de composé en vrac à environ 1 bar (15 psig) pour initier la filtration ;
k. filtrer la solution de composé en vrac sur un filtre stérilisant dans un récipient récepteur stérile refroidi en continu, présentant une température de 0 ° à 5 °C ; et
l. remplir en conditions aseptiques de la formulation pour intraveineuse des flacons en verre stérile, et sceller partiellement les flacons avec des bouchons de lyophilisation stérilisés secs ;
m. lyophiliser la formulation par congélation dans les flacons de verre à une température de -45° à -40 °C pour produire une formulation congelée ;
n. effectuer un séchage primaire de la formulation congelée à une température de -25 ° à -15 °C pendant 48 à 60 heures à une pression d'environ 0,1 mbar (80 mTorr);
o. effectuer un séchage secondaire de la formulation à une température de 40 ° à 60 °C à une pression d'environ 0,1 mbar (80 mTorr) ou moins, pendant environ 3 à 10 heures ;
p. refroidir les flacons à température ambiante ; et
q. sceller les flacons sous un vide partiel de 0,9 bar (700 Torr) ou moins, tout en maintenant une température d'environ 25 °C,
la formulation antibiotique de carbapénème stabilisé de formule II présentant une concentration de carbapénème d'environ 200 g/l et une teneur en carbonate d'environ 1 équivalent molaire.

13. Procédé selon la revendication 12, dans lequel le rapport des concentrations de composé en vrac à la solution d'hydroxyde de sodium est égal à 1 mole de composé en vrac/0,85 mole d'hydroxyde de sodium.

14. Procédé selon la revendication 13, dans lequel la solution lyophilisée présente une densité d'environ 1,11 g/ml.

15. Procédé selon la revendication 14 dans lequel, à l'étape j), la température dans l'appareil de malaxage, est d'environ 2 °C.

16. Procédé selon la revendication 15 dans lequel, à l'étape e, la température au moment de la dissolution du médicament en vrac dans la solution de bicarbonate de sodium, est de 2 °C.

17. Procédé selon la revendication 16 dans lequel, à l'étape 1, la température au moment du remplissage de la formulation dans les flacons, est de 0 ° à 5 °C.

18. Procédé selon la revendication 17, dans lequel la formulation stérile lyophilisée de l'étape o. présente une densité d'environ 1,11 g/ml à 5 °C.

19. Procédé selon la revendication 18, dans lequel la formulation de carbapénème stabilisé présente une concentration d'environ 200 g de carbapénème de formule II par litre de solution totale.
